# FASCICULE DE BREVET EUROPEEN

(11) **EP 3 568 688 B1**
(45) Date de publication et mention de la délivrance du brevet: **13.08.2025**
(21) Numéro de dépôt: 18705426.7
(22) Date de dépôt: 12.01.2018
(51) Int. Cl.: G01M 99/00, G01N 21/45, B05B 12/08, G01F 25/00, A61M 15/08

(54) **PROCÉDÉ D'ANALYSE D'UN SPRAY GÉNÉRÉ PAR UN DISPOSITIF DE DISTRIBUTION DE PRODUIT FLUIDE PHARMACEUTIQUE**
VERFAHREN ZUR ANALYSE EINES DURCH EINE VORRICHTUNG ZUR ABGABE EINES FLÜSSIGEN PHARMAZEUTISCHEN PRODUKTS ERZEUGTEN SPRAYS
METHOD FOR ANALYSING A SPRAY GENERATED BY A DEVICE FOR DISPENSING FLUID PHARMACEUTICAL PRODUCT

(30) Priorité: 16.01.2017 FR 1750318
(43) Date de publication de la demande: 20.11.2019
(73) Titulaire: Aptar France SAS, 27110 Le Neubourg (FR)
(72) Inventeur: BOISVILLIERS, Julien, 27110 Crosville La Vieille (FR)
(74) Mandataire: CAPRI
(86) Numéro de dépôt international: PCT/FR2018/050074
(87) Numéro de publication internationale: WO 2018/130791

(56) Documents cités:
- EP-A1- 3 047 912
- US-A1- 2013 257 966
- US-B2- 6 973 199
- BUCHMANN NICOLAS A ET AL: "A Novel High-Speed Imaging Technique to Predict the Macroscopic Spray Characteristics of Solution Based Pressurised Metered Dose Inhalers", PHARMACEUTICAL RESEARCH, 17 June 2014 (2014-06-17), Boston, pages 2963 - 2974, XP055905044, Retrieved from the Internet <URL:https://link.springer.com/content/pdf/10.1007/s11095-014-1391-6.pdf> [retrieved on 20220324], DOI: 10.1007/s11095-014-1391-6
- FAIVRE V ET AL: "Experimental and numerical investigations of jet active control for combustion applications", JOURNAL OF TURBULENCE, INSTITUTE OF PHYSICS PUBLISHING, BRISTOL, GB, vol. 5, no. 25, 5 August 2004 (2004-08-05), pages 1 - 16, XP001538482, ISSN: 1468-5248, DOI: 10.1088/1468-5248/5/1/025

## Description

La présente invention concerne un procédé d'analyse de spray généré par un dispositif de pulvérisation de produit fluide pharmaceutique.

Les dispositifs de pulvérisation de produit fluide pharmaceutique sont bien connus. En particulier dans des applications de pulvérisation nasale, l'efficacité thérapeutique du produit fluide pulvérisé peut dépendre des propriétés du spray généré lors de l'actionnement du dispositif. De manière connue, à la fin de la chaîne de montage, c'est-à-dire lorsque le dispositif de pulvérisation est assemblé, et juste avant d'être expédié chez le fabriquant du produit fluide pharmaceutique pour y être assemblé sur un réservoir correspondant, un certain nombre d'échantillons de dispositifs assemblés sont testés en laboratoire pour vérifier si les propriétés du spray correspondent au cahier des charges prédéfini.

Un inconvénient de ce système est qu'il concerne des dispositifs assemblés, et donc destructeur de ces dispositifs qui ne pourront plus, après avoir été testés, être livré au client. De plus, ce système impose une vérification humaine des dispositifs testés, et n'est donc pas totalement automatisable.

Le document « Assessment of fuel spraying using schlieren system » de Marek Klimkiewicz, Agriculture No 65, Vol. 2015, p. 119-126, décrit l'utilisation de la strioscopie pour analyser des sprays de carburant issus de différents injecteurs de carburant. Le document EP 3 047 912 A1 divulgue des moyens de détection qui comprennent au moins un capteur thermographique pour capturer au moins une image de la différence de température entre au moins une partie d'un fluide nébulisé éjecté d'un distributeur et un espace de contraste et/ou la projection de ladite image sur ledit espace de contraste.

La présente invention a pour but de surmonter les inconvénients susmentionnés.

En particulier, la présente invention a pour but de fournir un procédé d'analyse de spray qui soit non destructeur des dispositifs testés.

La présente invention a également pour but de fournir un procédé d'analyse de spray qui soit automatisé en grande partie.

La présente invention a également pour but de fournir un procédé d'analyse de spray qui est simple et/ou peu coûteux à fabriquer, à assembler et à utiliser.

La présente a donc pour objet un procédé d'analyse d'un spray généré par un dispositif de pulvérisation de produit fluide pharmaceutique tel que décrit dans la revendication 1. Des modes de réalisation avantageux sont décrits dans les revendications dépendantes. Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique d'un dispositif pour analyser un spray, selon un premier mode de réalisation avantageux,
- la figure 2 est une vue schématique d'un dispositif pour analyser un spray, selon un second mode de réalisation avantageux,
- la figure 3 est une vue schématique similaire à celle de la figure 2, d'une variante de réalisation avantageuse,
- les figures 4 à 6 sont des vues schématiques montrant des images obtenues par un procédé selon l'invention,
- la figure 7 est une vue schématique d'un spray de fluide test conforme, obtenue par un procédé selon l'invention, et
- la figure 8 est une vue similaire à celle de la figure 7, montrant un spray de fluide test non conforme.

Un objectif de l'invention est d'améliorer la qualité du contrôle des pulvérisateurs. Pour cela, l'invention prévoit d'utiliser la strioscopie pour analyser de manière automatique le cône du spray émis par des dispositifs pulvérisateurs.

La strioscopie, ou méthode dite de Schlieren, est une méthode optique de visualisation qui permet d'isoler dans une image les détails et petites variations, notamment des faibles variations d'indice comme lors de la compression de l'air ou d'autres fluides. D'une manière imagée, l'idée fondamentale de cette méthode est de retirer la lumière qui n'a pas été déviée par l'objet, par exemple le fluide étudié. En effet seuls les rayons déviés par celui-ci correspondent à des turbulences ou fréquences spatiales hautes en optique. Pour réaliser cela, on réalise d'abord une image de la source de lumière, de préférence une source de lumière incohérente, par exemple à l'aide d'une lentille convergente. À l'endroit précis de l'image géométrique passent les rayons qui n'ont pas subi de déviation (fréquences spatiales nulles). On les élimine avec un filtre. Les autres rayons, qui ont été déviés, ne sont pas focalisés au même endroit et peuvent donc passer afin de former une image filtrée. En résumé, on élimine le fond continu de l'image et, conséquemment, les détails ou turbulences de l'objet, qui étaient « noyés » dans ce fond continu, deviennent apparents.

Le filtre utilisé peut être simplement un point, un fil de fer, ou une lame, par exemple du type "couteau de Foucault".

La strioscopie est une application du filtrage spatial en optique de Fourier. En effet, la diffraction de Fraunhofer nous indique que la lentille crée, dans son plan focal image, la transformée de Fourier de l'objet en question. On visualise ainsi, dans ce plan, les fréquences spatiales associées à l'objet, et le filtre est placé dans ce même plan afin d'éliminer certaines de ces fréquences spatiales. Cette interprétation ondulatoire de la strioscopie la rend comparable à un filtrage passe-haut.

Dans le cadre de la présente invention, l'objectif est la mise en évidence d'un flux de fluide test, à savoir un jet d'air à température différente de la température ambiante, par exemple de l'air chauffé, issu de l'orifice de pulvérisation 2 d'une tête de pulvérisation 1 d'un dispositif de pulvérisation de produit fluide pharmaceutique, et son observation au travers d'une caméra. En variante, on peut aussi utiliser un jet d'air à température inférieure à la température ambiante.

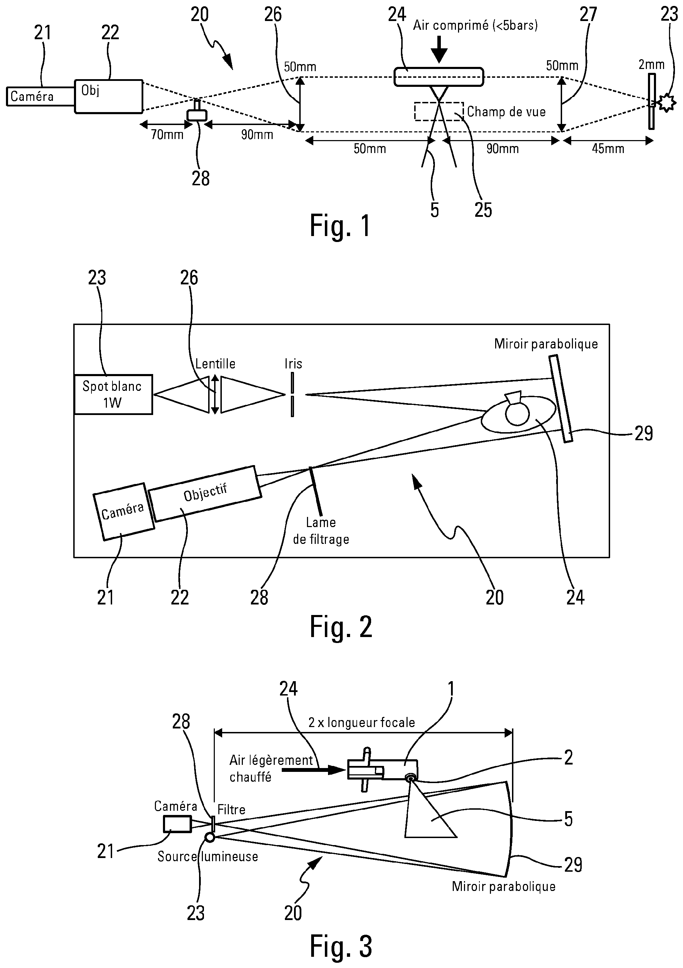

La figure 1 montre un banc strioscopique 20 selon un premier mode de réalisation avantageux.

Dans cet exemple, une caméra 21 associée à un objectif 22 est disposée d'un côté du banc 20 et une source de lumière 23 est disposée du côté opposé. Des moyens de génération d'air comprimé 24 sont prévus pour faire passer un flux d'air, de préférence à une température différente de la température ambiante, à travers une tête de pulvérisation 2, disposée dans une zone de visualisation 25, disposée entre deux lentilles de collimation 26, 27. Une lame ou diaphragme 28 est prévue devant l'objectif 22 pour couper le faisceau et ainsi filtrer l'image, et ainsi visualiser le spray, tel qu'expliqué ci-dessus.

Les composants suivants, de type standards, peuvent être utilisés pour cette mise en œuvre:

| Composats | Descriptif |
|---|---|
| Camera | Manta 1292x964 @ 30Hz |
| Objectif | 85mm f:1.8 |
| Lentille collimation | 50mm |
| Lentille collimation | 50mm |
| Lampe | Led blanche |
| Diaphragme | 2mm |
| Impulsion | Smartek 10µs, 12A |

Les figures 2 et 3 montrent deux variantes d'une autre mode de réalisation avantageux, dans lesquelles le banc strioscopique comprend un miroir concave et/ou parabolique 29. Ce mode de réalisation est plus compact, avec la source lumineuse 23 et la caméra 21 disposées du même côté, et le miroir 29 disposé du côté opposé. Le fonctionnement global est similaire au banc de la figure 1.

La visualisation comprend des vidéos.

Il est à noter que les images obtenues par la visualisation du spray peuvent comprendre, en plus, des images statiques (photos).

Pour réaliser l'analyse des sprays visualisés, l'invention comporte des moyens d'analyse 30 pour déterminer si le spray de fluide test issu de ladite tête de pulvérisation est conforme ou non conforme à des spécifications prédéterminées.

Ces moyens d'analyse 30 peuvent notamment comprendre des moyens de mesure de l'angle d'ouverture du spray de fluide test.

Eventuellement, des moyens de traitement d'image des visualisations de spray de fluide test peuvent être utilisés pour réaliser ladite analyse.

Ainsi, les spécifications prédéterminées comprennent un angle d'ouverture de spray prédéterminé, de sorte que les sprays dont l'angle d'ouverture est supérieur ou égal audit angle d'ouverture de spray prédéterminé sont classés conformes et les sprays dont l'angle d'ouverture est inférieur audit angle d'ouverture de spray prédéterminé sont classés non conformes.

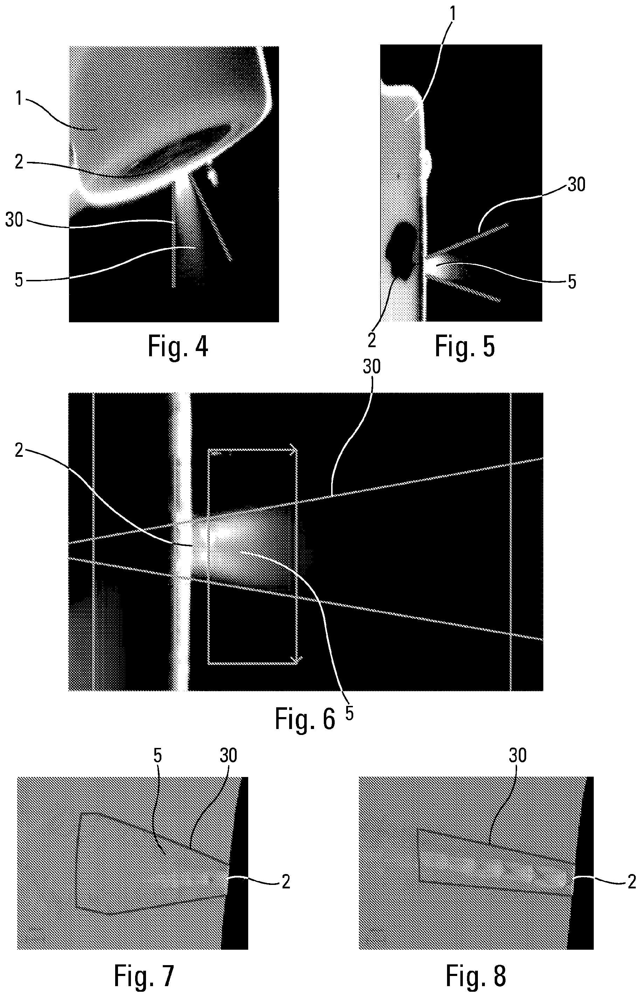

Les figures 4 à 6 illustrent des images obtenues avec le procédé et le dispositif de l'invention, sur lesquelles il est possible de mesurer et/ou d'évaluer l'angle d'ouverture du spray, tel qu'illustré par les traits coniques.

Les figures 7 et 8 illustrent des images montrant respectivement un spray de fluide test classé conforme (figure 7) et un spray de fluide test classé non conforme (figure 8).

La présente invention présente de nombreux avantages, et notamment:
- elle permet un contrôle automatisé du spray sur divers types de dispositif de pulvérisation;
- elle permet une analyse non destructive desdits dispositifs de pulvérisation;
- elle utilise un montage compacte et facilement adaptable;
- elle utilise des composants simples et standards, donc généralement non coûteux;
- elle permet un traitement d'image robuste, qui peut être réalisé en temps réel;
- elle assure une bonne répétabilité et une bonne discrimination des sprays conformes et non-conformes.

La présente invention a été décrite en référence à divers modes de réalisation avantageux, mais il est entendu qu'un homme du métier peut y apporter toutes modifications, sans sortir du cadre de la présente invention tel que défini par les revendications annexées.

## Revendications

1. Procédé d'analyse d'un spray généré par un dispositif de pulvérisation de produit fluide pharmaceutique, comprenant les étapes suivantes:
- fournir une tête de pulvérisation (1) d'un dispositif de pulvérisation de produit fluide pharmaceutique, ladite tête de pulvérisation (1) comportant un orifice de pulvérisation (2),
- faire passer un fluide test à travers ladite tête de pulvérisation (1) en direction dudit orifice de pulvérisation (2), ledit fluide test étant de l'air à température différente de la température ambiante,
- visualiser par strioscopie le flux de fluide test sortant dudit orifice de pulvérisation (2), et
- analyser ladite visualisation du flux de fluide test pour déterminer si le spray de fluide test issu de ladite tête de pulvérisation est conforme ou non conforme à des spécifications prédéterminées,
dans lequel ladite visualisation comprend des vidéos,
dans lequel lesdites spécifications prédéterminées comprennent un angle d'ouverture de spray prédéterminé, de sorte que les sprays de fluide test dont l'angle d'ouverture est supérieur ou égal audit angle d'ouverture de spray prédéterminé sont classés conformes et les sprays de fluide test dont l'angle d'ouverture est inférieur audit angle d'ouverture de spray prédéterminé sont classés non conformes.

2. Procédé selon la revendication 1, dans lequel ladite étape d'analyser comprend de déterminer l'angle d'ouverture du spray de fluide test.

3. Procédé selon la revendication 1 ou 2, dans lequel ladite étape d'analyser comprend une étape de traitement d'image de ladite visualisation du flux de fluide test.

## Patentansprüche

1. Verfahren zur Analyse eines von einer Vorrichtung zum Sprühen eines fluiden pharmazeutischen Produkts erzeugten Sprays, das die folgenden Schritte umfasst:
- Bereitstellen eines Sprühkopfs (1) einer Vorrichtung zum Sprühen eines fluiden pharmazeutischen Produkts, wobei der Sprühkopf (1) eine Sprühöffnung (2) aufweist,
- Leiten eines Testfluids durch den Sprühkopf (1) in Richtung der Sprühöffnung (2), wobei das Testfluid Luft mit einer von der Umgebungstemperatur abweichenden Temperatur ist,
- Visualisieren des aus der Sprühöffnung (2) austretenden Testfluidstroms mittels Strioskopie, und
- Analysieren der Visualisierung des Testfluidstroms, um festzustellen, ob das aus dem Sprühkopf austretende Testfluidspray den vorgegebenen Spezifikationen entspricht oder nicht,
wobei die Visualisierung Videos umfasst,
wobei die vorbestimmten Spezifikationen einen vorbestimmten Sprayöffnungswinkel umfassen, so dass Testfluidssprays, deren Öffnungswinkel größer oder gleich dem vorbestimmten Sprayöffnungswinkel ist, als konform klassifiziert werden und Testfluidsprays, deren Öffnungswinkel kleiner als der vorbestimmte Sprayöffnungswinkel ist, als nicht konform klassifiziert werden.

2. Verfahren nach Anspruch 1, wobei der Analyseschritt das Bestimmen des Öffnungswinkels des Testfluidsprays umfasst.

3. Verfahren nach Anspruch 1 oder 2, wobei der Analyseschritt einen Bildverarbeitungsschritt der Visualisierung des Testfluidstroms umfasst.

## Claims

1. A method of analyzing a spray generated by a spray device for spraying pharmaceutical fluid, comprising the following steps:
- providing a spray head (1) of a spray device for spraying pharmaceutical fluid, said spray head (1) including a spray orifice (2),
- causing a test fluid to pass through said spray head (1) towards said spray orifice (2), said test fluid being air at a temperature that is different from ambient temperature,
- displaying, by strioscopy, the flow of test fluid leaving said spray orifice (2), and
- analyzing said display of the test-fluid flow so as to determine whether or not the test-fluid spray coming from said spray head complies with predetermined specifications,
wherein said display comprises videos,
wherein said predetermined specifications include a predetermined spray cone angle, such that test-fluid sprays having a cone angle that is greater than or equal to said predetermined spray cone angle are classed as being compliant, and test-fluid sprays having a cone angle that is less than said predetermined spray cone angle are classed as being non-compliant.

2. A method according to claim 1, wherein said analyzing step includes determining the cone angle of the test-fluid spray.

3. A method according to claim 1 or claim 2, wherein said analyzing step includes an image-processing step for processing said display of the test-fluid flow.
